# EUROPEAN PATENT APPLICATION

(11) **EP 4 837 593 A1**
(43) Date of publication of application: **11.11.2026**
(21) Application number: 25736117.0
(22) Date of filing: 02.01.2025
(51) Int. Cl.: A61K 49/22, C01B 33/152, A61K 9/00

(54) **METHOD FOR INTEGRATING NANOBUBBLES AND NANODROPS OF OXYGEN INTO HYDROGELS FOR BIOLOGICAL AND REGENERATIVE MEDICINE APPLICATIONS**

(30) Priority: 02.01.2024 CL 2024000007
(71) Applicant: Cells For Cells S.A., Santiago 7550101 (CL)
(72) Inventor: VIAFARA GARCÍA, Sergio, Santiago, 7550101 (CL); ACEVEDO COX, Juan Pablo, Santiago, 7550101 (CL)
(74) Representative: Elzaburu S.L.P.
(86) International application number: PCT/CL2025/050002
(87) International publication number: WO 2025/145262

(57) **Abstract**

Method to stably incorporate oxygen nanobubbles and/or nanodroplets into hydrogels for biological and regenerative medicine applications where gas micro/nanobubbles (MNBs) and/or micro/nanodroplets (MNDs) are formed by compression and extension cycles in a closed system, by vacuum degassing cavitation before hydrogel polymerization. The hydrogel formed and its use as: biological support for tissue regeneration/generation; biological matrix as a support for cells, for cell invasion; to supply oxygen, and optionally active components such as: medications, growth and/or migration factors, antibodies, peptides, vesicles, cell derivatives and/or oligonucleotides, to tissues.

## Description

### TECHNICAL FIELD

The invention relates to the field of hydrogels, especially the field of hydrogels that serve biological and regenerative medicine applications. For example, hydrogels that serve as scaffolds for living cells, such as mesenchymal stem cells, where these scaffolds can be used in tissue regeneration, for example. The invention allows for obtaining porous hydrogels with a gas supply, such as oxygen, in the form of micro/nanobubbles or micro/nanodroplets within the hydrogel, where the gas can be transferred to the cells contained in said hydrogel or scaffold.

### BACKGROUND TO THE INVENTION

Replacement of damaged tissues and organs through regenerative medicine and tissue engineering is considered the best treatment for many chronic diseases or critically sized injuries. In the context of the shortage of organ and tissue donors, the development of new tissues or functional organs is a priority issue for public health. Tissue engineering has emerged as an ideal technology for this endeavor. In simple terms, tissue engineering provides a matrix or scaffold with healthy cells and inserts them into the tissue to be recovered, allowing these new cells to regenerate damaged tissue.

Tissue engineering must consider several transcendental factors for its use in therapy, highlighting the role of scaffolding, and other biological components, capable of mimicking the native environment of cells and replacing damaged tissue with the highest fidelity of copy or bioinspiration. In this context, scaffold design must consider physical and biological aspects of tissues for proper signaling towards cell maintenance, differentiation, and tissue regeneration. In the case of physical aspects, we highlight porosity and stiffness as aspects that contribute to the mechanobiology of tissues. In terms of biological factors, biocompatibility, cell infiltration, cell adhesion, encapsulation or binding of growth factors, and incorporation of helper cells can also modulate to improve the performance of biomaterials to repair, replace, or induce regeneration of injured tissues and organs.

Scaffolds are three-dimensional arrangements of biomaterials capable of mimicking the native extracellular matrix (ECM) and facilitating cell expansion and/or the pattern or arrangement of different cell types, matrix deposition, and tissue production. In this sense, one of the most promising biomaterials are hydrogels. They are composed of a hydrophilic polymer network capable of absorbing water molecules through hydrogen bonds, polar and ionic interactions. Hydrogels can mimic ECM and be used in biomedical applications such as drug delivery and tissue engineering.

In some tissue engineering applications, hydrogels are formed from a liquid composition of polymers and cells suspended in solution. This prior composition or pre-gel is cross-linked through irreversible mechanisms such as exposure to UV light, generating cross-linking of the matrix by free radical polymerization (FRP). However, the inventors noticed that the scaffolds of hydrogels polymerized by FRP generate an extremely hypoxic environment, with values close to anoxia. This could compromise cell viability, proliferation, mitochondrial function and other functions in regeneration, in addition to the risk of cell death during the avascular phase of biomaterial implantation, mainly in the central zone of the hydrogel.

Delivering oxygen effectively in tissue engineering can be challenging due to limitations in 1*) the diffusion of hydrophilic molecules and*/*or gases, 2) prevascular phase*, *3) oxygen consumption*, *4) scaffold design,* and *5) biodegradation rates.* Studies have shown that oxygen delivery occurs through passive diffusion from the surrounding environment, which leads to insufficient oxygen delivery in deeper layers of tissue, resulting in cell death and compromised tissue functionality. In addition, oxygen diffusion within implanted tissues is limited to approximately ~200 µm [Jain, R et al: Nat Biotechnol 2005, 23, 821-823], resulting in severe oxygen deficiencies in the nucleus of implanted tissues. Therefore, implant vascularization is essential to overcome limitations in oxygen diffusion, but this is a slow process, with only 5 mm of vascular penetration in 25 days for implanted porous scaffolds [Mikos, A. G et al: Biotechnology and Bioengineering 1993, 42(6), 716-723], during which period implant viability must be maintained.

Cell survival during the early stages of implantation, especially during the prevascular stage, is a major challenge in translational applications of tissue engineering (TE) and 3D bioprinted tissues. Therefore, supplementing oxygen (O₂) to the transplanted cells through an O₂ supply source such as micro/nanobubbles (MNBs) and/or micro/nanodroplets (MNDs), capable of acting as a reservoir of therapeutic agents and oxygen molecules. This is a promising approach to overcome low oxygen, mitigate hypoxia-related cell death, and improve cell viability.

In recent years, various materials have been developed that offer a gradual and constant release of oxygen over time [Oh, S. H et al: Biomaterials 2009, 30(5), 757-762.; Gholipourmalekabadi, M., et al: Trends in biotechnology 2016, 34(12), 1010-1021]. While oxygen-releasing biomaterials (ORBs) can help improve oxygen availability, it remains a challenging task to develop efficient and durable oxygen-releasing materials. Finding the right balance between sustained oxygen release and biocompatibility is a significant challenge.

To solve this technical problem, the present invention combines the use of micro/nanobubbles and micro/nanodroplets in synthetic or natural biopolymers, preferably in hydrogels generated by crosslinking the matrix by polymerization by free radicals, such as Methacrylate Salmon Gelatin (sGelMA) hydrogels. In this way, a new formulation of ORB is provided, capable of mitigating hypoxic stress in cells immobilized in hydrogels, an aspect that had not been sufficiently evaluated until now and that currently constitutes a technical limitation in tissue engineering. This problem is magnified especially in hydrogels formed by polymerization with free radicals, since as we indicated, periods of hypoxia/anoxia are generated when the hydrogel is exposed to UV light and activates the polymerization process.

This new integration of biomaterials together with micro/nanobubbles and micro/nanodroplets of oxygen allows the polymers to be provided with a hybrid composition for the supply of oxygen and the generation of pores in the constructs (hydrogels).

### DESCRIPTION OF THE FIGURES

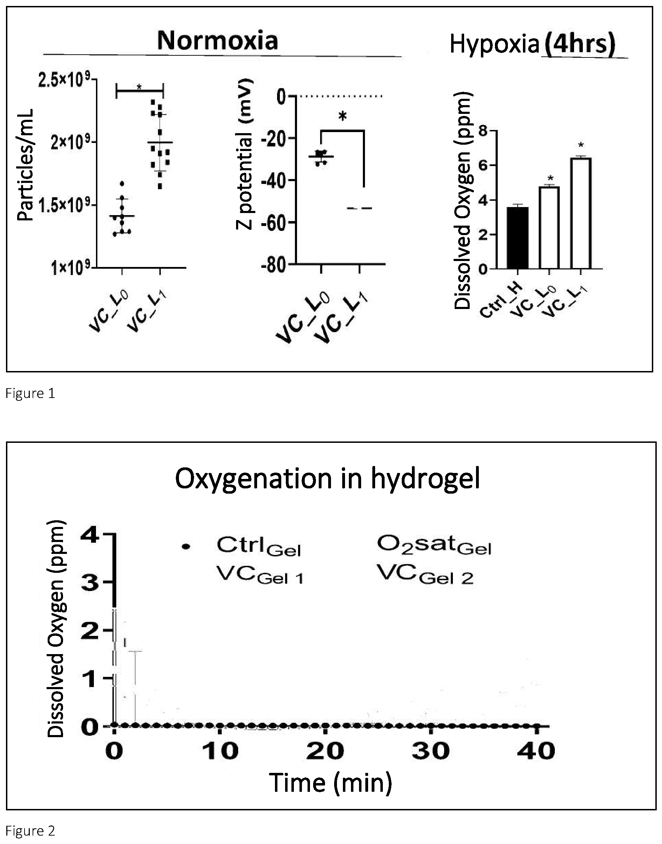

FIG. 1 shows the result of the formation of MNBs in culture medium in the presence VC_L1 and absence VC_L0 of surfactant, as explained in example 1. In the Normoxia condition, the number of nanobubbles per mL and the Z potential of both compositions according to the invention are plotted. In the hypoxia condition, a diagram is seen that shows characterization of the composition of the invention (ViaCox_Liquid) according to ISO/TC 281 and its oxygen levels during 4 hours of hypoxia. Significance p≤0.05 (One-way Anova) Three independent experiments in triplicate n=3. VC_L0 (medium + oxygen MNBs). VC_L1 (medium + oxygen MNBs + surfactant).
FIG.2 is a diagram showing the levels of dissolved oxygen in hydrogels polymerized by Free Radicals with UV light and the ability of the composition of the invention to mitigate or eliminate anoxia in these hydrogels after polymerization. Ctrl Gel (hydrogel control), VC_Gel1(Formulation with MNBs + Surfactant). VC_Gel2 (Formulation with MNBs + surfactant + oxygen MNDs), O₂ Sat Gel (hydrogel control saturated with oxygen).

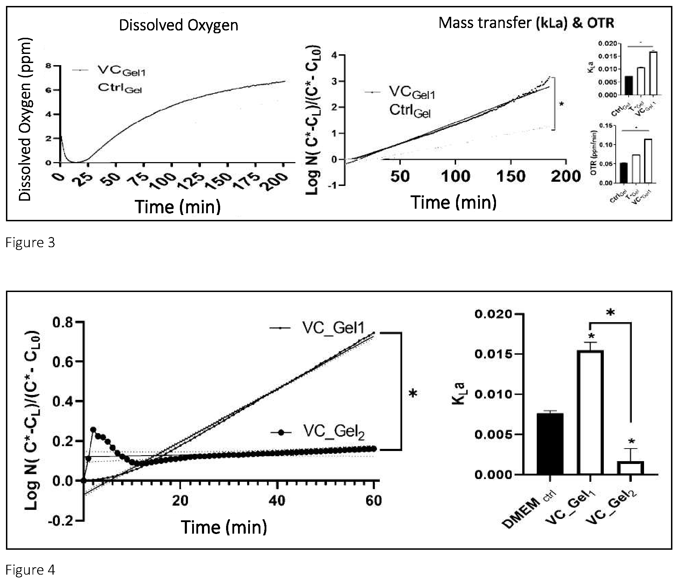

FIG. 3 is a diagram showing the oxygen transfer coefficients in hydrogels developed with the composition of the present invention, showing greater release and rapid oxygen transfer compared to the control. Significance p≤0.05 (unpaired T-tests, One-way Anova). Three independent experiments in triplicate n=3. Control_Gel (Hydrogel Control). ViaCox_Gel1 (formulation with oxygen + surfactant MNBs).
FIG. 4 is a diagram showing the oxygen transfer coefficients in hydrogels developed with the composition of the present invention, showing differential coefficients in oxygen transfer (fast-release hydrogels or more controlled-release oxygen hydrogels. Significance p≤0.05 (unpaired T-tests, One-way Anova). Three independent experiments in triplicate n=3. Control_Gel (hydrogel control). ViaCox_Gel1 (formulation with oxygen + surfactant MNBs). ViaCox Gel2 (formulation with oxygen MNBs + surfactant + oxygen MNDs + PFC [1% v/v]).

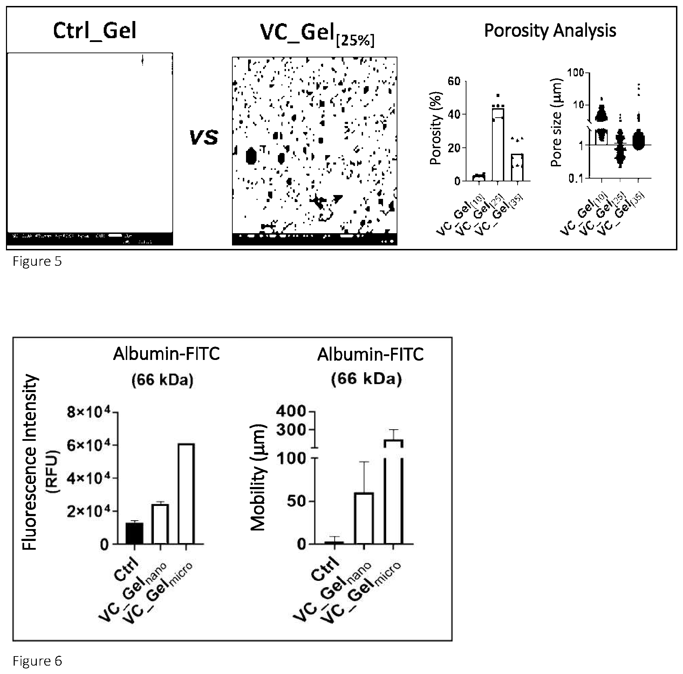

FIG. 5 is a graph showing the porosity in hydrogels manufactured with the composition of the present invention. Significance p≤0.05 (One-way Anova). Three independent experiments in triplicate n=3. Ctr_Gel (Hydrogel control). ViaCox Gel2 (Hydrogel (10, 25, 35% v/v) + MNBs + surfactant + MNDs).
FIG. 6 is a diagram showing the mobility of albumin conjugated to the anionic fluorophore FITC as an indirect marker of diffusivity in hydrogels manufactured with the composition of the present invention. Significance p≤0.05 (Unpaired T-tests, One-way Anova). Three independent experiments in triplicate n=3. Ctrl (Gel control without MNBs or MNDs). ViaCox Gel1ₙₐₙₒ (nanobubble-enriched hydrogel). ViaCox Gel1_{micro} (hydrogel enriched in microbubbles).

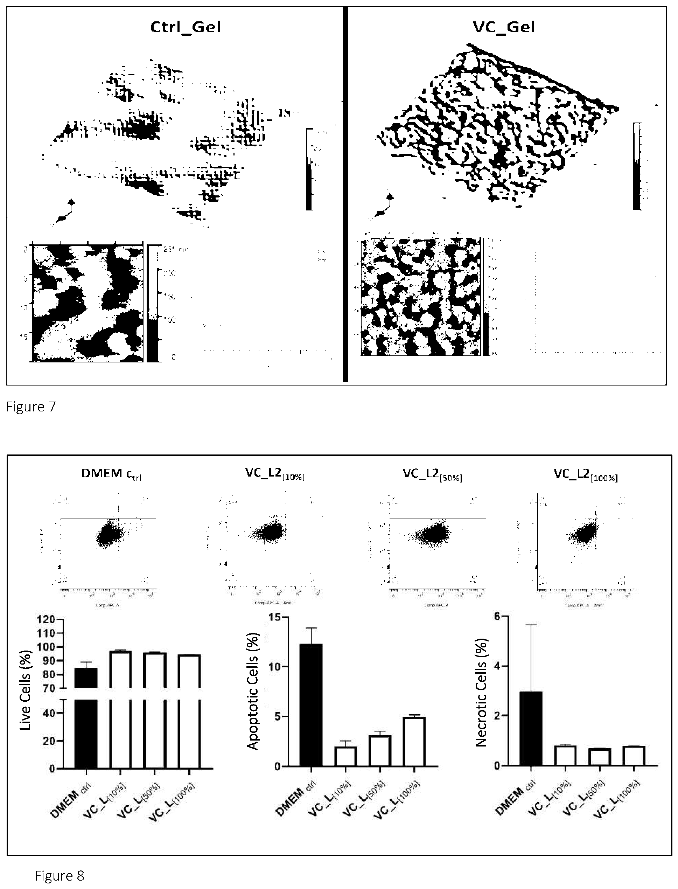

FIG. 7 is a diagram showing the effect of the composition of the present invention on the topography and nanoroughness of hydrogels. Ctr_Gel (hydrogel only). VC_Gel2 (hydrogel with MNBs + Surfactant + MNDs).
FIG. 8 is a diagram showing improvements in MSC cell survival and its reduction in apoptosis and necrosis after prolonged hypoxia of 3 days in cultured mesenchymal cells with the composition of the present invention DMEM_{ctrl}(Medium alone). VC_L 2_{[10%]} (Medium with MNBs+MNDs at 10% v/v). VC_L 2_{[50%]} (Medium with 50% MNBs+MNDs)_{.} VC_L 2_{[100%]}(Medium with 100% MNBs+MNDs).

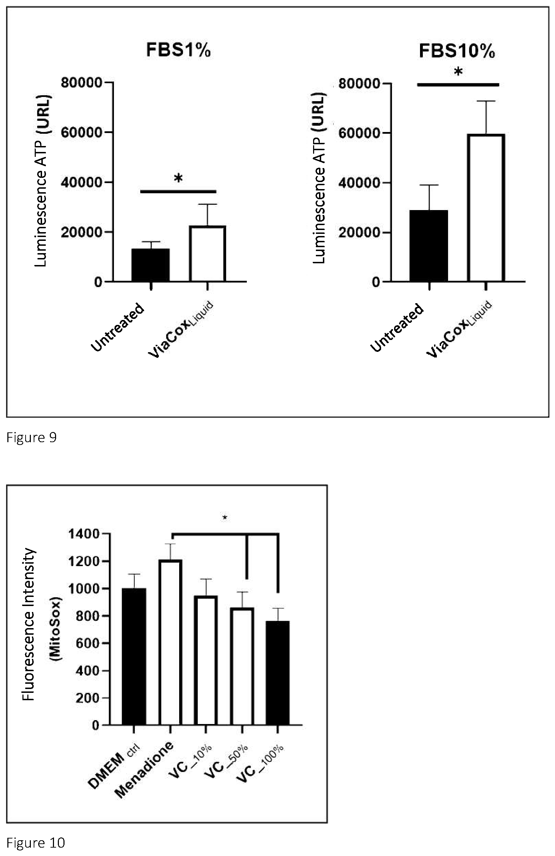

FIG. 9 is a diagram showing ATP levels when growing MSC cells with the composition of the present invention. Significance p≤0.05 (unpaired T-tests) Three independent experiments in triplicate n=3. Untreated (half solo). ViaCox_Liquid (medium + oxygen MNBs + Surfactant). RLU (Relative Light Unit)
FIG. 10 is a diagram showing the antioxidant action (reducing mitochondrial ROS levels) of the composition of the present invention on MSC cells. Significance p≤0.05 (One-way Anova) Three independent experiments. N=3. DMEM _{ctrl}(Medium alone). Menadione (positive mitochondrial stress control). VC_L_{[10%]} (Medium with 10% v/v MNBs). VC_L_{[50%]} (Medium with 50% v/v MNBs)_{.} VC_L_{[100%]}(Medium with 100% v/v MNBs). Methacrylate Salmon Gelatin (sGelMA)Methacrylate Salmon Gelatin (sGelMA)

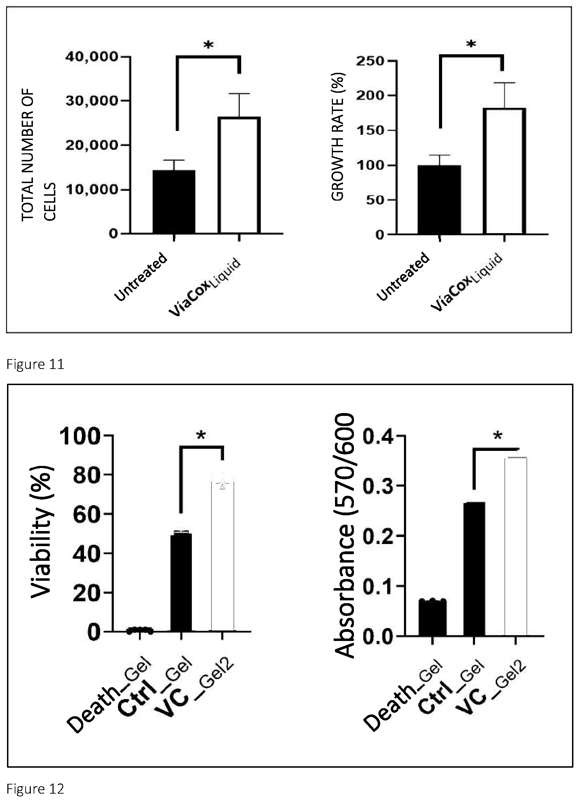

FIG. 11 is a graph in which the composition of the present invention favors the growth of MSC cells cultured in hypoxia (1%O₂). Significance p≤0.05 (unpaired T-tests) Three independent experiments in triplicate n=3. Untreated (half solo). ViaCox_Liquid (medium + oxygen MNBs + Surfactant).
FIG 12 is a diagram showing the increased viability and metabolic activity of cells grown in hydrogels exposed to FRP and hypoxia. Significance p≤0.05 (One Way Anova) Three independent experiments in triplicate n=3. Dead_Gel (Death Control Hydrogel). Ctrl_Gel (untreated hydrogel). VC_Gel2 (hydrogel with oxygen MNBs + Surfactant + oxygen MNDs).

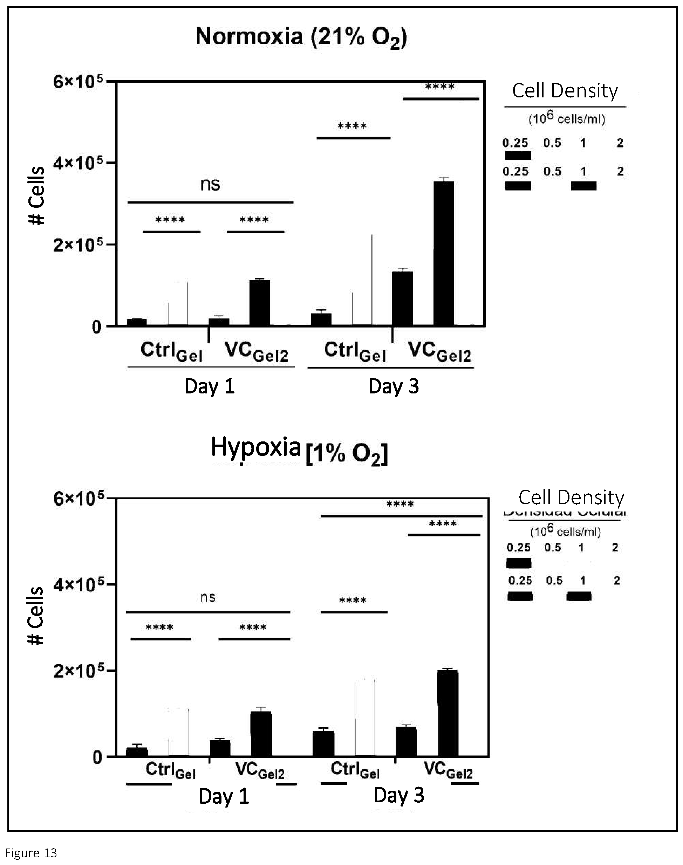

FIG 13 is a diagram showing the increase in proliferation of cells grown in hydrogels. The cultured cell density for each hydrogel was 0.25, 0.5, 1, 2 x10⁶/ml), then each hydrogel was cross-linked by UV light and incubated for 3 days at 37°C under conditions of normoxia and hypoxia, respectively. To determine the number of cells, a standard curve was previously determined at different cell densities. Significance p≤0.05 (One Way Anova) Three independent experiments in triplicate n=3. Ctrl_Gel (untreated hydrogel). VC_Gel2 (hydrogel with oxygen MNBs + Surfactant + oxygen MNDs)

### GENERAL DESCRIPTION OF THE INVENTION:

The present invention relates to a composition containing micro/nanobubbles (MNBs) and/or micro/nanodroplets of gases (MNDs), such as oxygen, and their use. More specifically, the present invention relates to a composition containing MNBs and MNDs in liquid solutions and in polymeric solutions; capable of forming matrices or hydrogels after a polymerization reaction. Where the composition of the invention is capable of supplying gases, such as oxygen and thus increasing the availability or solubility of this gas in both liquid fluids and hydrogels. Additionally, the composition of the invention is capable of modulating porosity, diffusivity and mechanical properties in hydrogels; In addition, the composition of the invention allows for increased solubility and availability of oxygen; and it allows mitigating cell deficiencies related to hypoxia; It also allows the activation of mitochondria contained in the hydrogel and/or in the cells found in the hydrogel and thus reduce mitochondrial stress.

In one embodiment, the invention reveals a composition to promote survival under hypoxic conditions and the growth of cultured cells by dissolving a gas in a liquid and in matrices using technology of MNBs and MNDs in hydrogels. The invention responds to the growing demand for the development of compositions, methods and products that require MNBs and MNDs and their use. The present invention also responds to the demand for a more efficient method to favor oxygen-releasing biomaterials (ORBs) under conditions of hypoxia and/or anoxia.

On the other hand, mitochondria are energy-producing organs, and it is believed that increasing mitochondrial activity can increase energy production and consequently increase cell proliferation. However, hydrogels that promote mitochondrial activity have not yet been developed, and there is a demand for the development of liquid and polymeric solutions that activate mitochondria and decrease hypoxic stress. The present invention makes up for this deficiency, since the composition of the invention allows the mitochondria within the hydrogel to be activated by maintaining a condition of normoxia within the hydrogel that contains the mitochondria.

According to this specification, the following invention is provided.
(1) A composition containing MNBs with a size in (a) liquid (ViaCox_Liquid) of 200-400 nm and a composition of MNBs + MNDs + biopolymers + photoinitiator in (b) hydrogels (ViaCox_Gel) of 90 micrometers or less. The use of liquid-phase MNBs contains 10-12 ppm of oxygen, while the combination of MNBs and MNDs for hydrogels contains 18-22 ppm of oxygen. Here, the gas concentration is the concentration of both the dissolved gas and the gas contained in the MNBs and MNDs.
(2) a composition in liquids (ViaCox_Liquid) or hydrogels (ViaCox_Gel) defined in (1a) or (1b) capable of increasing cell survival.
(3) a composition in liquids (ViaCox_Liquid) or hydrogels (ViaCox_Gel) defined in (1a) or (1b) capable of decreasing cell death.
(4) a liquid or hydrogel composition defined in (1a) or (1b) capable of increasing ATP production.
(5) a liquid or hydrogel composition defined in (1a) or (1b) capable of reducing mitochondrial stress due to hypoxia.
(6) a hydrogel composition defined in (1b) capable of providing higher concentration of oxygen in matrices.
(7) a hydrogel composition defined in (1b) capable of mitigating anoxia by free radical polymerization (FRP) during hydrogel formation.
(8) a hydrogel composition defined in (1b) capable of providing oxygen transfer coefficients to mitigate anoxia by free radical polymerization (FRP) during hydrogel formation.
(9) a hydrogel composition defined in (1b) capable of providing differential coefficients in oxygen transfer (fast-release hydrogels or more controlled oxygen release hydrogels) to mitigate anoxia by free radical polymerization (FRP) during hydrogel formation.
(10) a hydrogel composition defined in (1b) capable of retaining oxygen during exposure to UV light, thereby avoiding free radical polymerization (FRP) anoxia during hydrogel formation.
(11) a hydrogel composition defined in (1b) capable of generating pores in constructs (hydrogels)
(12) A hydrogel composition defined in (1b) capable of increasing the diffusion coefficient of gases and water-soluble molecules.
(13) a hydrogel composition defined in (1b) capable of generating differential topographies with respect to the control in terms of nano-roughness.
(14) A hydrogel composition defined in (1b) capable of improving survival in mesenchymal cells encapsulated in hydrogels and cultured in hypoxia.
(15) A hydrogel composition defined in (1b) capable of enhancing proliferation in mesenchymal cells encapsulated in hydrogels and cultured in hypoxia.

Depending on the composition of the present invention employed, various effects can be obtained on the properties of formed liquids and hydrogels, the composition of the invention being capable of improving oxygenation, porosity, diffusion or mechanical properties of the liquid or hydrogel: In addition, the composition of the invention has an effect on the cellular response of the cells that are in contact with said composition, by favoring metabolic activation, increasing ATP production, promoting cell proliferation and reducing cell damage or death in seeded cells: in 2D through composition (1a) and in 3D through composition (1b), during hypoxic conditions.

### METHODOLOGY AND IMPLEMENTATION:

The invention points to aqueous or polymeric solutions with micro/nanobubbles (MNBs) and micro/nanodroplets of gases (MNDs), such as oxygen, capable of acting as an oxygen-releasing biomaterial (ORB), useful to be used in reactions for the formation of biopolymers, hydrogels, or other matrices that act as scaffolds for living cells.

Although one of the preferred embodiments is in hydrogels, the scope of the present invention is not limited to these, since the technology can also be extended to different types of biopolymers, natural, synthetic and/or their combinations. As well as different methods of functionalizations and/or polymerization strategies, the latter whether physical, chemical, reversible or irreversible, among others. In a preferred embodiment, the composition of the invention is used in the formation of hydrogels, and in a particularly preferred embodiment, methacrylate hydrogels.

Regarding the use of the present invention in polymeric solutions, emphasis is placed on the use of the composition formulated according to the invention to generate hydrogels with oxygen MNBs+MNDs embedded in a biological and/or synthetic matrix with different types of functionalizations and/or with different polymerization strategies.

The composition of the invention comprises a solution that in turn includes a polymer, either natural or synthetic, with different types of functionalizations, and/or with different polymerization strategies. Although the hydrogels submitted by the inventors relate to polymers of methacrylate salmon gelatin (sGelMA), the scope of the present invention is not limited by this single type of hydrogels, nor this single type of functionalization or polymerization.

The polymers in the composition of the invention may be natural or synthetic and may or may not be functionalized. Where functional groups include but are not limited to amine groups, carboxylic acid, biotin, azide, thiol, hydrazide, alkyne, succinic ester, vinyl, folate, methacrylate, styrene, acrylonitriles, vinyl-ether, vinyl-ester, maleimide, vinyl-ketone, acrylamide, isocyanate, etc. Compositions with these polymers conveniently also have polymerization initiator compounds, such as photoinitiators. These can be chosen between both types of photoinitiators, classified according to the type of dissociation before UV light (Norrish type 1 and Norrish type 2). The polymerization initiator is usually an oxidizing agent. Polymerization initiators also include those that are activated by exposure to electromagnetic radiation or heat.

Polymerization initiators can also be used, which are described, for example, in the publication of US patent application No. US2010/0137241, or any other known in the art.

The composition and general stages of the manufacturing method in polymeric solutions exemplified in the present invention and the development of the formulation are described below.

In a realization, to obtain a composition in accordance with the invention requires:
i) providing a liquid composition consisting of a gelatin with a low melting point, less than 15°C, determined by the fact that it has a proline and hydroxyproline content of 18% or less with respect to the total amino acid content; which is additionally functionalized with methacryloyl or methacrylamide groups; and a photoinitiator;
ii) adding a surfactant (T) and a perfluorocarbon compound (PFC) directly in the composition defined in i in a proportion or ratio between 0.001 to 0.1% v/v for the surfactant and a ratio of between 0.5 and 10% v/v for the PFC compound.
iii) transfering the composition defined in ii to a closed tube or container (capable of insulating from atmospheric pressure) and allowing the injection of high-purity oxygen at controlled pressure.
iv) forming oxygen MNBs+MNDs by compression and extension cycles in a closed system (vacuum degassing cavitation) obtaining MNBs+MNDs directly in the composition defined in ii
v) initiating the polymerization of the liquid dispersant phase by application of light radiation; and
vi) obtaining a hydrogel with micro/nanobubbles + micro/nanodroplets of oxygen embedded in a matrix or hydrogel.

Preferably, the invention proposes the use of fluorinated compounds such as fluorostensiplus + liquid PFC as a liquid mixture of colloidal stability and suspended in sGelMA in both solution and hydrogel. In this way, we seek the integration of micro/nanodroplets +micro/nano-immiscible bubbles in 3D scaffolding, promoting not only oxygen availability in tissue engineering, but also gaining porosity properties, a key parameter to promote better nutrient diffusion and gas coefficients.

For the composition of the invention, an agent capable of offering high efficiency in the reduction of surface tension is required, which is an important aspect in the field of bubble stability and bioprinting, being a relevant requirement to create three-dimensional (3D) structures of living cells and biomaterials. Therefore, the preferred surfactant for this invention is anionic, fluorinated and branched that translates into a fluorosurfactant.

A preferred embodiment is the commercial fluoro-surfactant Tivida^{®} FL 2300, the first commercially available surfactant C2-Fluoro of the Tivida^{®} FL technology platform, which exhibits favorable properties not only for its ionic and structural (branched) nature, but also for its exceptional reduction of surface tension for a wide spectrum of liquid phases, offering several additional advantages such as the absence of oral toxicity, zero aquatic toxicity, lack of bioaccumulation.

On the other hand, the second fluorinated compound in the composition is preferably perfluorocarbon (PFC). Although in the background the PFC has already stood out in biomedicine for its ability to function as possible artificial substitutes for blood due to its chemical and biological inertance, ease of sterilization and high solubility in oxygen. PFCs are a type of oxygen transporter with a high capacity to solubilize oxygen (20 times greater than that of water). This remarkable oxygen-carrying property allows PFCs to be used in artificial blood. PFCs have also been applied during tissue culture to increase the survival of mesenchymal stem cells (MSCs) under hypoxic conditions. However, PFCs alone have a short retention time *in vivo* due to their low molecular weight, which limits their applications in ischemic tissue regeneration, so it was not obvious to employ them in the present invention.

There is very little literature available on the use of biomaterials combined or embedded with micro/nanodroplets and micro/nanobubbles, and the need for this resource is what motivated the inventors to develop the present invention.

In one embodiment, the invention consists of the development of a formulation capable of integrating MNBs+MNDs that, suspended in polymeric matrices + fluorosurfactant + liquid PFC, allow for an increase in the availability of oxygen in matrices to mitigate deficiencies related to hypoxia, thus improving cell survival in the short/moderate term under hypoxic and/or anoxic conditions.

Specifically, both surfactants and PFC compounds are used under the conditions provided by the manufacturer, which is used in the proportions already indicated, i.e. liquid fluorosurfactant (T) is used in a proportion of between 0.001 to 0.1% v/v, preferably between 0.005 and 0.05. And the liquid perfluorocarbon compound (PFC) is used in a ratio of between 0.5 and 10% v/v, preferably between 1 and 5% v/v. For example, fluorinated perfluorodecalin can be used as a PFC, which the manufacturer provides as a 100% liquid solution with a density of 1.93 g/cm3. Tivida FL 2300^{®} can be used as a surfactant, where the manufacturer provides a stock solution at 33% w/v of the active component, anionic fluorosurfactant. and with a density of 1.05 gr/cm³. This fluorosurfactate stock is prepared on the solvent 1-methoxy-2-propanol.

The composition of the present invention is produced by converting dissolved oxygen gas containing micro/nanobubbles or micro/nanodroplets into liquid solutions (e.g., ultrapure water, culture medium, polymeric solutions). Although the purity of the gas is not specifically limited, when using it for drinking water, it is preferable to use high-purity gas.

The production of the present invention consists of a method of preparation for non-polymeric and polymeric liquid solutions, containing MNBs, MNDs, or MNBs-MNDs either in colloidal suspension of liquid phases or confined within semi-solid phases such as polymeric matrices or hydrogels, capable of oxygenating, generating pores, increasing diffusivity, modulating crosslinking density, nanotopography and mechanical properties of the matrix.

In the present invention, the nanobubble generator can be used without particular limitation as long as it is a conventional nanobubble generator. For example, the present invention is obtained from a preparation method that requires a liquid fluid for use in a Venturi (2a) or vacuum degassing cavitation (2b) system, the two-phase working fluid has a liquid phase and a gas phase, comprising:
- a fluid inlet;
- a fluid outlet;
- at least one gas inlet;
- And fine bubble generators or a combination of bubble generators 2A and 2B described below. 2a and 2b.

### 2a. Venturi Method

- Fine bubbles can be generated in a pulsating manner using a mechanical fluid pulsation controller to manipulate the inlet fluid or fluid outlet of the fine bubble generator system.
- The gas and liquid are mixed in a generator component called a "Venturi tube or valve", which includes a gas inlet, then the mixed gas-liquid flows promoting various loops in a Venturi valve and promoting the collapse of the bubbles to form micro and nanobubbles (MNBs).
- The subsequent overflow of cavitation is released into a container and recycled or recirculated again throughout the system.
- Microbubbles and nanobubbles (MNBs) can be present in the fluid at different time periods.
- This method allows working with large quantities of liquids (1L and 25L).
- The inlet water pressure can be as low as 0.08 Mpa, no additional gas needs to be introduced, and an ambient air inlet is sufficient. The number of fine bubbles can be up to 100,000,000 per mL.
- The pressure source for the liquid can be a pump, which provides the energy needed to circulate the fluid.
- The operating pressure has a minimum of approximately 0.08 M Pa, with a preferred range of approximately 0.2 M Pa to approximately 0.5 M Pa.

### 2b Vacuum or vacuum degassing method

- For small volumes (less than 7ml), the production of the compositions of the invention called ViaCox_Liquid or ViaCox Gel were preferably generated by the vacuum degassing method, from standardized parameters such as compression and extension cycles (60 cycles), and fluoro-surfactant (Tivida^{®} 0.02%).
- A syringe is filled with pure water or another liquid, and after expelling any air trapped inside, the tip of the syringe is sealed with a Luer lock cap.
- The water or other liquid is subjected to vacuum inside a syringe at a constant temperature of 20°C, creating a gas desaturation that, when followed by the release of the vacuum, leads to the formation of micro/nanobubbles (MNB) and micro/nanodroplets (MND).
- The water or other liquid is then depressurized by rapidly pulling the plunger on the syringe and then re-pressurized by instantaneous release of the plunger, which travels at a relatively high speed under the action of vacuum pressure (typically ~7 cm s-1). This is relevant for biomedical and in vitro research applications. For example, the generation of MNBs+MNDs for the engineering of oxygen-releasing matrices, especially for injectable and porous bio-inks. This bimodal platform of promoting oxygenated and porous hydrogels is mainly feasible by this invention, promoting greater diffusivity and modulation of the surface nanotopography and mechanical properties of hydrogels.
- Regarding the advantages of this method, there is the fact that it shortens its production times, the versatility to work with different biopolymers and surfactants at small, low and moderate volumes, and it is also a clean, scalable, automatable and efficient method in terms of quantity, quality and stability of the MNBs+MNDs.

In this way, the invention refers to a method to stably incorporate gas nanobubbles and/or nanodroplets into aqueous solutions for biological and regenerative medicine applications, which includes the following stages:
i) Providing an aqueous liquid composition of functionalized or non-functional, biologically compatible natural or synthetic polymers and a polymerization initiator compound;
ii) Adding directly to the composition defined in i (a) or (b):
   a liquid fluorosurfactant (T) in a ratio of 0.001 to 0.1% v/v; and
   a liquid perfluorocarbon compound (PFC) in a ratio of 0.5 to 10% v/v;
iii) taking the composition obtained in ii to a closed tube or container (capable of insulating from atmospheric pressure) and allowing oxygen to be injected at controlled pressure;
iv) forming micro/nanobubbles (MNBs) and/or micro/nanodroplets (MNDs) of gas by means of compression and extension cycles in a closed system, by cavitation by vacuum degassing obtaining MNBs and MNDs directly in the composition defined in ii,
v) obtaining polymeric aqueous solution with stable micro/nanobubbles and/or micro/nanodroplets of oxygen;
vi) initiating the polymerization of the solution either by reversible polymerization (physical) and/or by irreversible polymerization (chemical or photochemical); and obtaining a hydrogel with stable micro/nanobubbles and/or micro/nanodroplets of oxygen embedded in a matrix or hydrogel, providing porosity and availability of oxygen within the hydrogel.

Where the anionic surfactant is a branched fluorosurfactant; and oxygen is high-purity oxygen.

The liquid composition also includes active compounds such as medications, growth and/or migration factors, antibodies, peptides, vesicles, cell derivatives and/or oligonucleotides; and/or additionally comprises living components such as cells or organelles; where the organelle is an active mitochondria.

In one embodiment, the polymeric liquid composition is a gelatin with a low melting point, less than 15°C, determined because it has a proline and hydroxyproline content of 18% or less with respect to the total amino acids, which is functionalized with methacryloyl or methacrylamide groups, in a proportion of 30% to 100% of lysine residues of the amino acid chain of said gelatin. Specifically, low-melting point gelatin is derived from organisms of the genus Salmo or Oncorhynchus.

Where the polymerization of stage vi) is initiated by UV radiation in the presence of a photoinitiator at a concentration between 0.01% and 5% (w/v).

In a second aspect, the invention points to the hydrogel formed according to the method described above, which is formed of gelatin polymerized with oxygen nanobubbles in a concentration of 10⁶ to 10⁹ micro/nanobubbles (MNBs) and/or micro/nanodroplets (MNDs) per cm³, which gives it porosity and oxygen availability. Conveniently the oxygen is high purity oxygen.

This hydrogel of the invention additionally includes active compounds such as medications, growth and/or migration factors, antibodies, peptides, vesicles, cell derivatives and/or oligonucleotides; and/or additionally comprises living components such as cells or organelles. Where the organelle is an active mitochondria.

In a third aspect, the invention points to the use of hydrogel as a biological support for tissue regeneration/generation. Where tissue regeneration is in a joint injury, cartilage injury, bone injury, ulcer injury, ligament injury, organ injury, spinal cord injury, or soft tissue injury. Or conveniently tissue regeneration is in a reconstitution of skin tissue, muscle tissue, soft tissue, or post-surgical replacement tissue.

In an additional aspect, the invention points to the use of hydrogel as a biological matrix as a support for cells, for cell invasion.

The invention is also useful in traumatic or blood perfusion blockade events involving inflammation and/or ischemia of skin tissue, musculoskeletal tissue, vascular tissue, nerve tissue, brain tissue, spinal cord, liver tissue, pancreatic tissue, intestinal tissue, lung tissue, cardiac tissue, to supply oxygen and active components. For example, the hydrogel of the invention can be placed, for example in a surgical procedure, on tissue damaged by a blood perfusion blockage, in order to rapidly supply oxygen and active components for tissue preservation.

Finally, the invention also points to the use of hydrogel as an acellular biological matrix, biological matrix as a mechanical support, biological matrix for active components.

The present invention will be described below by reference to examples, but the scope of the present invention is not limited by these examples.

### EXAMPLES

### Example 0.

Obtaining composition for hydrogel.

First, purified and filtered salmon gelatin was obtained which was then dried and crushed.

The crushed salmon gelatin was dissolved to a desired final concentration of 10% to 35% (w/v) in PBS 1X (pH 7.4) at 60°C. Once completely dissolved, while still stirring, methacrylic anhydride (276685, Sigma, USA) was slowly added to a final concentration of 8% (v/v). Different levels of methacryloyl and/or methacrylamide functionalization require different concentrations of methacrylic anhydride. After 3 h of reaction, a 5X dilution in 1X PBS was performed and the reacted gelatin was dialyzed against deionized water at 40 °C for 1 week. Daily replacements of fresh deionized water were performed to remove all unreacted methacrylic anhydride during dialysis. Finally, the dialysate mixture was filtered with 20 µm porous filter paper, freeze-dried, and stored at -20 °C for later use.

With this process, the base biomaterial, methacrylate salmon gelatin (sGelMA) is obtained at different concentrations. This is then combined with a photoinitiator, such as Irgacure^{®} 2959 [BASF Resins], preferably in a concentration of between 0.01% and 5% (w/v), which is activated with UV light to form a hydrogel. The concentration used for photoinitiator was 0.5% w/v.

For the validation of the composition of the ViaCox Liquid, or ViaCox_L invention , different conditions were evaluated in the following examples, which have the following components:
Control Liquid: ultrapure water, PBS or culture medium
ViaCox_Liquid_0: ultrapure water, PBS or culture medium + uncoated oxygen MNBs.
ViaCox_Liquid_1: ultrapure water, PBS or culture medium + surfactant-coated oxygen MNBs
ViaCox_Liquid_2: ultrapure water, PBS or culture medium + surfactant-coated oxygen MNBs + oxygen MNDs and PFCs [1-5%].
(PBS: Phosphate-buffered saline)

For the validation of the composition of the invention ViaCox Gel, ViaCox_G, different conditions were evaluated in the following examples, which have the following components:
Control: sGelMA (10% v/v) + 0.1% photoinitiator,
ViaCox_Gel0: sGelMA (10 % v/v), 0.1% photoinitiator, uncoated oxygen MNBs.
ViaCox_Gel1: sGelMA (10% v/v), 0.1% photoinitiator, 0.02% v/v surfactant-coated oxygen MNBs.
ViaCox Gel2: sGelMA (10% v/v), 0.1% photoinitiator, 0.02% v/v surfactant-coated oxygen MNBs, oxygen MNDs and PFCs [1-5% v/v]

### Example 1:

The method preferentially used in the present invention is based on Henry's law principle and the use of vacuum outgassing, and is evaluated in this example.

In liquid culture media, micro/nanobubbles coded as ViaCox_L₀ (or VC_L₀) and ViaCox_L₁ (or VC_L₁) were generated, as defined in 2b, with the generation of MNBs being the last process of production. Regarding ViaCox_L₂ is described later in example 8.

Specifically, as indicated in 2b, 5 mL of previously oxygenated DMEM medium (for 2 minutes) was taken either oxygenated medium alone (ViaCox_L₀ ) and/or with Tivida 0.02% v/v (ViaCox_L_{!} ) in a syringe, once any air trapped inside was expelled, the tip of the syringe was sealed with a Luer locking cap. The syringe was connected to a vacuum degassing cavitation system, comprising a fluid inlet; a fluid outlet; and a gas inlet. The system has a constant controlled temperature at 20°C. Each test, ViaCox_L₀ and ViaCox_L1, underwent 60 cycles of compression and extension. In the case of ViaCox_L₁, oxygen was combined with the fluoro-surfactant Tivida^{®} at a concentration of 0.02% (v/v)

Both compositions showed the presence of billions of nano entities by NTA and with favorable colloidal stability to remain in suspension by zeta potential.

It is observed that ViaCox_L1 is the composition with the highest production (2.0x10^9 mL-1 bubbles) and zeta potential (-51.9 mV) compared to the 1.4x10^9 mL-1 bubbles and -27.9 mV. The results are plotted in Figure 1, for Normoxia. It is observed that the condition of the method of the invention VC_L₁ forms more nanobubbles with a lower z-potential.

Additionally, the effect of VC_L was tested, favoring a higher level of oxygenation in liquids subjected to hypoxia or oxygen-deficient environments [1% O₂] for 4 hours at 37°C Figure 1, for Hypoxia. This shows that the nanobubbles formed are stable in a liquid solution, where the condition of the invention has significantly greater stability in an aqueous solution, such as a culture medium.

### Example 2:

Oxygen dynamics were measured simultaneously with UV polymerization of a hydrogel.

As we have indicated, it is known that the polymerization of hydrogel by UV under normal conditions generates a highly anoxic environment. Therefore, the ability of the nanobubbles of the invention's method to remain intact during the polymerization process will be evaluated.

For this, 5 mL compositions were prepared according to what is indicated in example 0, for ViaCox Gel: Control, VC_Gel1, VC_Gel2 and oxygen-saturated control. 5ml of the liquid of interest of each of the compositions was added to a 50ml falcon tube, this tube was connected by a natural thin wall tubin #30 to an adapter that connects with a 99.9% oxygen cylinder. This connection allowed oxygen to be carried from the cylinder to the medium or liquid with an oxygen flow of 0.5 L/min, at an oxygen pressure of 0.05 Mpa, and for 2 min. As indicated, VC_Gel1, includes Tivida 0.02% surfactant, and VC_Gel2 comprises Tivida 0.02%v/v surfactant and PFC [1%v/v]

As indicated, polymerization with UV light was initiated, and the concentration of dissolved oxygen in the different compositions was measured up to 400 minutes after polymerization. The results are shown in Figure 2.

Both compositions of the invention evaluated, VC_Gel 1 and 2, maintained dissolved oxygen in the biopolymer matrix after polymerization. Where VC_Gel 1 contributed to reducing the anoxic period, and ViaCoxGel2 showed the best protection from anoxia, after UV-mediated free radical polymerization.

Interestingly, the oxygen-saturated sGelMA does not show any difference from the sGelMA control without prior oxygenation, in both cases no dissolved oxygen was detected after polymerization. This reflects that oxygen saturation alone is not enough to improve oxygen levels after UV polymerization. Consequently, it is suggested that the presence of ViaCox_Gel1 contributes to the shorter anoxia window, while ViaCoxGel2 showed the best anoxia protection, thus mitigating the anoxic period in its entirety in sGelMA hydrogels.

### Example 3:

The oxygen transfer coefficients in hydrogels developed with the composition of the present invention, specifically ViaCox_Gel1, were studied.

Hydrogels with branched fluoro-surfactant (ViaCox_Gel1) stabilized nanobubbles were shown to be 5 times more efficient in reducing the anoxic period than the sGelMA control. (Fig. 3). The experimental determination of the oxygen transfer coefficient was carried out following the gassing-out method or elimination of gases as a consequence of exposing functionalized hydrogels with methacryl groups to UV light (365 nm). For the purpose of generic observations, in this first example we will dispense with a cellular encapsulation (acellular evaluation), so we will apply the gas removal technique we will also consider the model of methacrylicized hydrogels and the degassing shown in the previous example (anoxia due to exposure of functionalized gelatin to ultraviolet light or blue light.

Due to the restriction posed for the development of the measurement, equation (1), which refers to the oxygen transfer rate (OTR). Equation 1: $OTR = kLa ⋅ \left(C∗-C\right) = kLaΔC .$*C*= oxygen saturation concentration in liquid, C = oxygen concentration in liquid.*

The terms *kL*, and *a* are a difficult to measure directly; it is usually evaluated as *kLa*, called the volumetric transfer coefficient. Understanding that equation (1) only shows the variation of C, over time, an integration between the limits to t = o and C, =o gives the following equation: Equation 2: $\begin{matrix}Ln \left(\begin{matrix}C ∗ - C \\ C ∗ - CL 0\end{matrix}\right) = - kLa ⋅ t , where Ln = Natural logarithm , CLO = concentration of oxygen in liquid \\ time zero .\end{matrix}$*where Ln = Natural logarithm, CLO = concentration of oxygen in liquid time zero*

Therefore, Eq.(2) is a line of natural logarithm Ln(1 -C/C*ql vs t with slope equal to - *kLa*. The experimental measurements needed to evaluate *kLa* should consist of variations of C, (t). We propose to use this method in a hydrogel in which, prior to any test, the oxygen dissolved in the hydrogel is eliminated by exposure to UV light (20 seconds) by bubbling an inert gas, nitrogen, until c, =O.

This data confirms that the presence of MNBs allows for a significantly higher K*_{L}a* value, which correlates with a greater ability to facilitate oxygen transfer (OTR) from the nanobubble gas core to the liquid phase (Fig. 3).

### Example 4:

The oxygen transfer coefficients in hydrogels developed with the composition of the present invention, ViaCox_Gel1 and ViaCox_Gel2, were studied.

As indicated, we developed a second formulation of the invention by incorporating perfluorocarbon (PFC) with the aim of improving the polymer's affinity for oxygen. These compositions were called ViaCox_Gel2.

As shown in example 2, the development of hydrogels ViaCox_Gel2 significantly reduced the anoxic period compared to ViaCox_Gel1 (Fig. 2). In addition, 2 ppm of DO ViaCox_Gel2 shown inside the hydrogel and remained constant throughout the oxygen release experiment (60 minutes). These results demonstrate that the ViaCoxGel2 composition protects against anoxia, generating a greater balance in oxygen levels, showing a more controlled and continuous mass transfer coefficient, which is related to a more leisurely or slower oxygen release compared to the ViaCoxGel1 composition. Figure 4 shows the oxygen transfer coefficients (OTRs) for Control, ViaCox_Gel1, and ViaCox_Gel2.

This remarkable difference between K_{L}a (mass transfer in a liquid phase) and OTR suggests that ViaCox_Gel2 has a slower oxygen transfer than ViaCox_Gel1, which implies that when the hydrogels are used with the formulations of the invention, they will be oxygenated for longer periods and have coefficients of slow and rapid oxygen release.

### Example 5:

The design of the present invention combines the use of micro/nanobubbles and micro/nanodroplets in natural polymers such as Salmon Methacrylic-Gelatin (sGelMA). The objective of this strategy is to mitigate hypoxic stress in cells encapsulated in sGelMA matrices, an aspect that has not yet been evaluated or addressed and that currently represents a technical limitation in tissue engineering, especially in hydrogels formed by free radical polymerization. Simultaneously, this invention also contributes to the development of polymers with a hybrid composition (micro/nanobubbles and micro/nanodroplets) not only for a greater and efficient delivery of oxygen and soluble factors, but also as a strategy for the generation of pores within the structures (hydrogels).

As for porosity, porosity is defined as the volumetric percentage of void spaces or pores within a material. In the context of tissue regeneration, especially in hard tissues (bone and cartilage), porosity and diffusion play a crucial role in facilitating the infiltration of cells, nutrients, and blood vessels, which are essential for tissue formation. The range of porosity and diffusion can vary depending on several factors, including the specific defect being treated, in general, for hard tissues, ~40% is an adequate range of porosity for proper regeneration.

The composition of the present invention increased the percentage of porosity in hydrogels with different concentrations of gelatin, or hardnesses according to analysis by SEM microscopy and ImageJ software. ViaCox Gel2 compositions with micro/nanobubbles and micro/nanodroplets with compositions with different concentrations of hydrogel or sGelMA, coded here as VC_Gel2 [10%], VC_Gel2 [25%], VC_Gel2 [35%], were obtained with the method of the invention, showed a wide range of up to 40% porosity as shown in Figure 5, with a pore size distribution between 210 nm and 90 µm.

### Example 6:

The composition of the present invention provides improvements in the diffusivity of hydrogels for soluble factors such as albumin conjugated to anionic fluorophores such as FITC. ViaCox_Gel shows higher fluorescence intensity and suggests greater diffusivity. The composition of this invention also showed greater distance traveled through the hydrogel, showing mobility of 2.6 -- 9.1 times greater than control hydrogels (Figure 6).

### Example 7:

Hydrogels manufactured with the composition of the present invention were scanned with Oxford's Jupiter XR AFM at room temperature using the resonance mode and a soft tip (AC240TS-R3). Micro/nano-entities compatible with MNBs or MNDs (ViaCox_Gel2) are observed that endow the hydrogels with different nano-roughness compared to the control and suggest their existence after gel polymerization (Figure 7). Therefore, the hybrid combination of micro/nanobubbles and micro/nanodroplets generates topographical differences compared to control hydrogels. These results are consistent with SEM (Fig. 5) and diffusion testing (Fig. 6). AFM allows for the presence of micro/nano bubbles and micro/nanodroplets to be confirmed in situ, as it does not require any additional processing compared to other microscopy techniques (e.g. SEM).

### Example 8:

In this experiment, umbilical cord mesenchymal cells were cultured in hypoxia [1%O₂] for 3 days and in the presence of medium + MNBs-MNDs (v/v) at different concentrations VC_L_2[10% v/v], VC_L_2[50%v/v], VC_L_2[100%v/v]. The results of the analysis for viability, apoptosis and necrosis are shown in Fig. 8, where all the formulations VC_L_2 evaluated improved the survival rate, decreased the rate of apoptosis and necrosis. The prolonged time of hypoxia shows a decrease in the viability of control cells, which reflects that even in hypoxic-resistant cells such as UC-MSCs, viability can begin to be affected after 3 days of hypoxia. However, cells exposed to increased oxygenation through ViaCox_L_2 composition showed significant improvements in promoting viability and decreasing apoptosis and necrosis.

### Example 9:

Serum deprivation and hypoxia are two common experimental conditions used in cell cultures to simulate specific physiological or pathological conditions for the study of cellular responses. Therefore, this patent focuses on conditions that mimic the reduced availability of oxygen and nutrients, as well as the growth factor limitations that cells may experience during certain physiological or pathological situations (e.g., tissue injury, ischemia, or specific stages of cell differentiation). Based on the above, the cells are treated with different concentrations of MNBs under low serum conditions (FBS 1%) and hypoxic conditions (1% O₂).

It was observed that under hypoxic conditions (1% O₂), VC_L1 can transport and supply oxygen to crops. Mesenchymal cells in the presence of ViaCox_L_1 significantly increasing the production of ATP (Fig. 9) independent of the percentage of availability of serum used in the medium. We found a 1.7- and 2.5-fold increase in ATP generation in hypoxia-FBS1% and hypoxia-FBS10%, respectively (Figure 9). All of the above suggests an important energetic effect on cell metabolism, mediated in part by greater oxygenation, which would favor greater mitochondrial function and, consequently, a metabolism more oriented to the participation of the mitochondria through the electron transport chain or OXPHOS metabolism.

### Example 10:

Experiment in which mitochondrial stress levels were measured in 2D UC-MSCs cultured in hypoxia for 24 hours (Fig. 10) by flow cytometry. Induction of hypoxia 1% O₂ increased mitochondrial ROS levels (880 UF) compared to untreated cells or in normoxia (466 UF). In addition, all UC-MSCs exposed to ViaCox_L1 decreased their ROS levels, showing a dose-dependent profile, which makes VC_L1 [100%] the most efficient composition in mitigating mitochondrial ROS (653 UF) and reaching values similar to untreated cells under normoxic conditions (data not shown). These results are the first evidence of the positive effect of ViaCox_L1 on the reduction of mitochondrial stress under 1% hypoxia.

### Example 11:

We measured the cell proliferation kinetics of 2D cultured UC-MSCs using metabolic indicators by colorimetry. The proliferation curve is mainly exponential during the first 3 days, with a maximum dish of 5 days. (Data not shown). We emphasize that only the hypoxic condition reveals a noticeable effect of the composition ViaCox_L1. Cells exposed to ViaCox_L1 (0.23 abs) during the expansion phase (days 1-3) achieved higher metabolic activity than untreated hypoxic cells (0.19 abs), but similar to untreated cells under normoxic conditions (0.24 abs). Subsequently, manual cell counting was performed after 24 hours of incubation (Fig 11), showing that the composition VC_L1 increases the total number of cells, as well as their growth rate. This result suggests that ViaCox_Liquid promotes 1.8-fold cell proliferation of UC-MSCs in hypoxia.

### Example 12:

For the biological and cellular evaluation in hydrogels, the cell density, the volume of the medium used and the concentration of FBS for the 3D culture were previously standardized (data not shown). Samples with different amounts of ViaCox_Gel2_con three different concentrations of PCFs [1, 2, 5%] were tested. We selected a DMEM volume of 400 µL per well as the best choice for future experiments. Hydrogels with VC_Gel2 significantly increased mitochondrial activity, but in a dose-dependent manner. According to the concentrations of PFCs evaluated [1%, 2.5%, 5%], levels of 0.27, 0.31, 0.35 abs were observed, respectively. The data in Fig 12 only shows the formulation with the highest metabolic activity compared to the control (0.19 abs), maintaining cell viability above 80% while cells encapsulated in control sGelMA showed 65% viability. Although these results are limited to short-term hypoxia (24 h, n=3), it is suggested that hydrogels manufactured with the composition of the present invention protect UC-MSCs against hypoxic stress, increasing their survival rate and promoting greater metabolic activity, the latter an indirect indicator of viability.

### Example 13:

To determine cell proliferation in 3D cultures, hydrogels were manufactured with the composition ViaCoxGel 2 of the present invention. After a follow-up of 3 days under conditions of Normoxia and hypoxia, a significant p≤0.05 effect (paired T-tests) was observed on cell proliferation for each of the cell densities evaluated (0.25, 0.5, 1, 2 x10⁶/ml). ViaCox _Gel2 being the most efficient condition to increase the rate of cell growth. This suggests that ViaCoxGel2 optimizes proliferation even in normoxia, demonstrating an obvious dual benefit, not only in its protective role in mitigating related deficiencies in hydrogels incubated in hypoxia[1% O₂], but also as an efficient hydrogel in increasing metabolic activity and cell number, even under normoxia conditions. This could favor future cell regeneration analyses. Significance p≤0.05 (paired T-tests) Three independent experiments in triplicate n=3. VC_Gel2[10% m/v]

### PERSPECTIVES/APPLICATIONS

The present invention creates fine and ultrafine bubbles and alternately nanodroplets in biopolymer-based constructs, highlighting the production method and potential use of biopolymers. In addition, the composition of the micro/nanobubbles with a gas core or micro/nanodroplets with a liquid PFC core shows enhanced affinity for oxygen and is able to reduce the anoxic period after free radical polymerization. This integration of micro/nanobubbles and nanodroplets with the various biomaterials gives the polymers a very useful composition for oxygen supply and porogenic purpose in constructs (hydrogels). In addition, the biopolymer-based constructs with fine bubbles and pores obtained by the present invention can be produced on a micro/nano scale, and it presents a structural configuration that would be adapted to meet mechanical and biofunctional requirements. In addition, these biopolymers would promote cell survival by reducing cell death induced by hypoxia or hypoxic stress after free radical polymerization; this is due to the increased availability of oxygen enclosed in the fine bubble/nanodroplet mixture.

The spectrum of applications based on MNBs and nanodroplets are very relevant for the development of natural or synthetic biopolymers sensitive to hypoxia capable of mitigating hypoxic stress and integrating the design of porous matrices simultaneously. In addition, this invention could have an impact on other fields of biomedicine, such as tissue engineering, the development of bioinks based on free radical polymerization, materials science, biomanufacturing, bioprinting along with the development of pharmaceuticals, organs-on-chips, organoids, cell therapy, regenerative therapy, among others.

## Claims

1. Method for stably incorporating oxygen nanobubbles and/or nanodroplets into hydrogels for biological and regenerative medicine applications **CHARACTERIZED in that** it comprises the following stages:
i) Providing an aqueous liquid composition of functionalized or non-functional, biologically compatible natural or synthetic polymers and a polymerization initiator compound;
ii) adding directly to the composition defined in i (a) or (b):
a liquid fluorosurfactant (T) in a ratio of 0.001 to 0.1% v/v; y
a liquid perfluorocarbon compound (PFC) in a ratio of 0.5 to 10% v/v;
iii) taking the composition obtained in ii to a closed tube or container (capable of isolating from atmospheric pressure) and allowing oxygen to be injected at controlled pressure;
iv) forming micro/nanobubbles (MNBs) and/or micro/nanodroplets (MNDs) of gas by means of compression and extension cycles in a closed system, by cavitation by vacuum degassing obtaining MNBs and MNDs directly in the composition defined in ii,
v) obtaining polymeric aqueous solution with stable micro/nanobubbles and/or micro/nanodroplets of oxygen;
vi) initiating the polymerization of the solution either by reversible polymerization (physical) and/or by irreversible polymerization (chemical or photochemical); and obtaining a hydrogel with stable micro/nanobubbles and/or micro/nanodroplets of oxygen embedded in a matrix or hydrogel, providing porosity and availability of oxygen within the hydrogel.

2. Method according to claim 1 **CHARACTERIZED in that** the anionic surfactant is a branched fluorosurfactant.

3. Method according to claim 1 **CHARACTERIZED in that** the oxygen is high-purity oxygen.

4. Method according to claim 3 **CHARACTERIZED in that** the liquid composition additionally comprises active compounds such as medications, growth and/or migration factors, antibodies, peptides, vesicles, cell derivatives and/or oligonucleotides.

5. Method according to claim 3 **CHARACTERIZED in that** the liquid composition additionally comprises living components such as cells or organelles.

6. Method according to claim 5 **CHARACTERIZED in that** the organelle is an active mitochondria.

7. Method according to claim 1 **CHARACTERIZED in that** the polymeric liquid composition is a gelatin with a low melting point, less than 15°C, determined by having a proline and hydroxyproline content of 18% or less with respect to the total amino acids, which is functionalized with methacryloyl or methacrylamide, in a proportion of 30% to 100% lysine residues of the amino acid chain of said gelatin.

8. Method according to claim 7 **CHARACTERIZED in that** low-melting gelatin is derived from organisms of the genus Salmo or Oncorhynchus.

9. Method according to claim 1 **CHARACTERIZED in that** the polymerization of stage vi) is initiated by UV radiation in the presence of a photoinitiator at a concentration between 0.01% and 5% (w/v).

10. Hydrogel formed according to the method of any one of claims 1 to 9, **CHARACTERIZED in that** it is formed of polymerized gelatin con Oxygen nanobubbles at a concentration of 10⁶ to 10⁹ micro/nanobubbles (MNBs) and/or micro/nanodroplets (MNDs) per cm³, which gives porosity and oxygen availability.

11. Hydrogel according to claim 10 **CHARACTERIZED in that** the oxygen is high purity oxygen.

12. Hydrogel according to claim 10 **CHARACTERIZED in that** it additionally comprises active compounds such as medications, growth and/or migration factors, antibodies, peptides, vesicles, cell derivatives and/or oligonucleotides.

13. Hydrogel according to claim 10 **CHARACTERIZED in that** it additionally comprises living components such as cells or organelles.

14. Hydrogel according to claim 13 **CHARACTERIZED in that** the organelle is an active mitochondria.

15. Hydrogel according to claim 10 **CHARACTERIZED in that** it serves as a biological support for tissue regeneration/generation.

16. Use according to claim 15 **CHARACTERIZED in that** tissue regeneration is in a joint injury, cartilage injury, bone injury, ulcerative injury, ligament injury, organ injury, spinal cord injury, or soft tissue injury.

17. Use according to claim 15 **CHARACTERIZED in that** tissue regeneration is in a reconstitution of skin tissue, muscle tissue, soft tissue, or post-surgical replacement tissue.

18. Use according to claim 15 **CHARACTERIZED in that** it serves as a biological matrix as a support for cells, for cell invasion.

19. Use according to claim 15 **CHARACTERIZED in that** it serves as an acellular biological matrix, biological matrix as a mechanical support, biological matrix for active components.

20. Use of the Hydrogel according to claim 10 **CHARACTERIZED in that** it serves in blood perfusion blockade or traumatic events involving inflammation and/or ischemia of skin tissue, musculoskeletal tissue, vascular tissue, nervous tissue, brain tissue, spinal cord, liver tissue, pancreatic tissue, intestinal tissue, lung tissue, cardiac tissue, to deliver oxygen and active components.
